# EUROPEAN PATENT APPLICATION

(11) **EP 4 624 876 A1**
(43) Date of publication of application: **01.10.2025**
(21) Application number: 25165215.2
(22) Date of filing: 21.03.2025
(51) Int. Cl.: G01H 11/08, G01N 29/036, G01N 29/24, G01N 29/32

(54) **SENSING DEVICE**

(30) Priority: 28.03.2024 JP 2024053814
(71) Applicant: Nihon Dempa Kogyo Co., Ltd., Tokyo 151-8569 (JP)
(72) Inventor: TSUCHIYA, Shoichi, Saitama (JP)
(74) Representative: Becker, Eberhard

(57) **Abstract**

A sensing device includes a sensor substrate, a Peltier element unit, a circuit board, a base body, a cover body, and a base cover. The Peltier element unit in contact with the sensor substrate. The base body includes a first recessed portion for housing a region of the Peltier element unit at a side of the heat dissipation surface, a second recessed portion for housing the circuit board, and a partition plate interposed between the first recessed portion and the second recessed portion. The cover body houses the base body in a region where the first recessed portion is formed, the Peltier element unit projecting outside the first recessed portion, and the sensor substrate, the cover body having an opening formed at a position corresponding to a region where the piezoelectric resonator is disposed for adsorption of the substance to be sensed.

## Description

### TECHNICAL FIELD

This disclosure relates to a sensing device that senses a substance to be sensed based on variation in frequency of a piezoelectric resonator.

### DESCRIPTION OF THE RELATED ART

A Quartz Crystal Microbalance (QCM) using a crystal unit is known as the sensing device that senses a substance contained in a gas. Among methods utilizing the QCM for substance analysis, there is a known method including the steps of adhering gas to the crystal unit, gradually raising the temperature of the crystal unit from the low-temperature state, and desorbing the gas adhered to the crystal unit.

This method as one type of thermogravimetry measures an amount of frequency variation between before and after desorption of the gas to acquire the gas adhesion amount. A component of the gas is identified by detecting a temperature at which the gas is desorbed. There is a known analyzer provided with a built-in Peltier element for actively changing the temperature of the crystal unit. The sensing device constituted by combining the QCM and the temperature control unit for desorbing the gas is called a Thermoelectric QCM (TQCM) (see Japanese Unexamined Patent Application Publication No. 2020-193846 as described below).

The TQCM provided with the temperature control unit and the oscillator circuit for the crystal unit has its structure complicated accompanied with size increase, resulting in the demand for size reduction.

### SUMMARY

### [Technical problem]

Japanese Unexamined Patent Application Publication No. 2005-257394 discloses a configuration constituted by stacking a crystal unit sensor, a Peltier element, and a heat transfer material from above in this order. Japanese Unexamined Patent Application Publication No. 2020-193846 discloses a configuration constituted by integrating a substrate provided with a piezoelectric resonator, a thermoelectric element unit, and a support plate, and detachably mounting the integrated configuration onto the base portion.

Since the size reduction is not considered by any of those documents, the configuration that overcomes the problem of the present disclosure is not disclosed in those documents.

A need thus exists for a sensing device which is not susceptible to the drawback mentioned above.

### [Technical solution]

According to an aspect of this disclosure, there is provided a sensing device that adsorbs a substance to be sensed as a gas to a piezoelectric resonator, and desorb the substance to be sensed by changing a temperature of the piezoelectric resonator to sense the substance to be sensed based on a relationship between a variation in an oscillation frequency of the piezoelectric resonator and the temperature. The sensing device includes a sensor substrate, a Peltier element unit, a circuit board, a base body, a cover body, and a base cover. The sensor substrate holds the piezoelectric resonator. The Peltier element unit is in contact with the sensor substrate. The Peltier element unit includes a temperature control surface for changing the temperature of the piezoelectric resonator and a heat dissipation surface opposite to the temperature control surface. The circuit board is electrically connected to the piezoelectric resonator, includes an oscillator circuit installed for oscillating the piezoelectric resonator and a connector that is surface-mounted for an external connection. The base body includes a first recessed portion for housing a region of the Peltier element unit at a side of the heat dissipation surface, a second recessed portion for housing the circuit board, and a partition plate interposed between the first recessed portion and the second recessed portion. The cover body houses the base body in a region where the first recessed portion is formed, the Peltier element unit projecting outside the first recessed portion, and the sensor substrate. The cover body has an opening formed at a position corresponding to a region where the piezoelectric resonator is disposed for adsorption of the substance to be sensed. The base cover covers the second recessed portion. The connector is externally connected from a side surface side of a region where the second recessed portion of the base body is formed.

According to another aspect of this disclosure, the Peltier element unit is housed in the first recessed portion to bring the heat dissipation surface into contact with the partition plate; and the circuit board is housed in the second recessed portion to cause the oscillator circuit to be apart from the partition plate.

According to another aspect of this disclosure, a separation distance between the oscillator circuit and the partition plate is in a range from 0.3 mm to 1.5 mm.

According to another aspect of this disclosure, the sensing device is used in a vacuum atmosphere; and a space between the second recessed portion and the base cover for housing the circuit board brought into the vacuum atmosphere causes the oscillator circuit disposed apart from the partition plate to be vacuum insulated from the partition plate.

According to another aspect of this disclosure, the connector is disposed between the partition plate and the circuit board; a thickness dimension of the partition plate in a region where the oscillator circuit is placed is larger than a thickness dimension of the partition plate in a region where the connector is placed.

According to another aspect of this disclosure, a thickness dimension of the partition plate in a region where the oscillator circuit is placed is in a range from 3 mm to 5 mm.

### [Technical effect]

The present disclosure provides the sensing device that senses the substance to be sensed adsorbed to the piezoelectric resonator based on variation in the oscillation frequency of the piezoelectric resonator. The sensing device includes the surface mounted connector for external connection onto the circuit board on which the oscillator circuit for oscillating the piezoelectric resonator is mounted. The circuit board is housed in the second recessed portion of the base body, and the connector is provided for external connection from the side surface side of the sensing device. This makes it possible to make the sensing device compact.

### BRIEF DESCRIPTION OF THE DRAWINGS

The foregoing and additional features and characteristics of this disclosure will become more apparent from the following detailed description considered with reference to the accompanying drawings, wherein:
FIG. 1 is a vertical cross-sectional side view of a sensing device according to a comparative configuration;
FIG. 2 is a perspective view of an outer appearance of the sensing device according to the comparative configuration;
FIG. 3 is a vertical cross-sectional side view of a sensing device according to an embodiment of the present disclosure;
FIG. 4 is an exploded perspective view of the sensing device according to the embodiment of the present disclosure;
FIG. 5 is a perspective view of an outer appearance of the sensing device according to the embodiment of the present disclosure;
FIG. 6 is a vertical cross-sectional side view illustrating an example of a sensing system provided with the sensing device according to the present disclosure; and
FIG. 7 is a vertical cross-sectional side view illustrating another example of the sensing device according to the present disclosure.

### DETAILED DESCRIPTION

The present disclosure relates to a sensing device formed as a TQCM, and aims at making the sensing device compact. Before describing the present disclosure, the configuration of the generally employed sensing device is described as a comparative configuration.

### <Comparative Configuration>

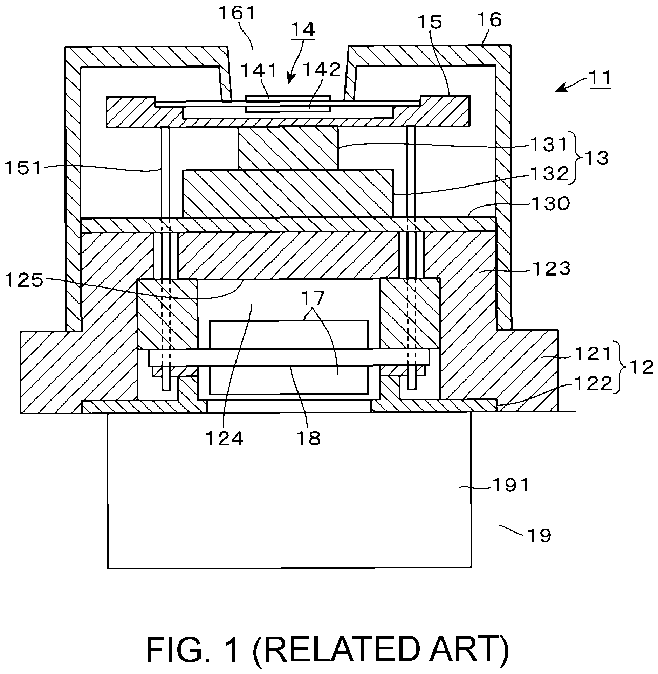

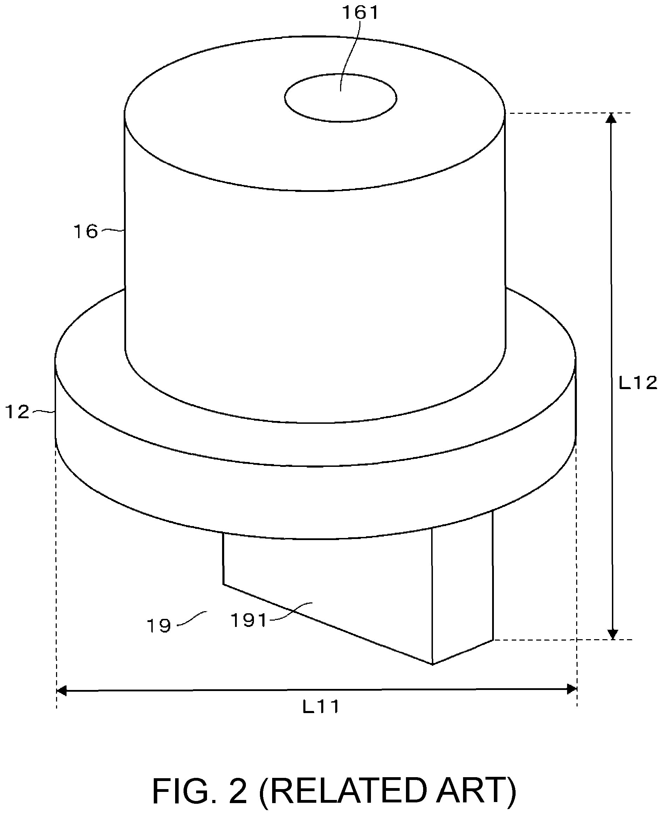

FIG. 1 is a vertical cross-sectional side view of a sensing device 11 according to the comparative configuration. FIG. 2 is a perspective view of an outer appearance of the sensing device according to the comparative configuration. The sensing device 11 as illustrated in the drawings includes a base portion 12 including a base main body 121 and a back lid 122. The base body 121 is constituted of a block having a circular shape in plan view. A protruding portion123 having a circular shape in plan view is provided on the center of the upper surface side of the base body.

A circular plate-shaped Peltier base 130 is provided on an upper surface of the base portion 12. A Peltier element unit 13 is disposed on the Peltier base 130. The Peltier base 130 has a recessed portion (not illustrated) for positioning the Peltier element unit 13 to be in place. A sensor substrate 15 including a crystal unit 14 is further provided on an upper surface side of the Peltier element unit 13. The crystal unit 14 includes excitation electrodes 141, 142 at both surfaces, which are electrically connected to an oscillator circuit 17 to be described later via conductive pins 151. The Peltier element unit 13 performs a temperature control on the crystal unit 14. In this example, prismatic-shaped Peltier elements 131, 132 that differ in size are vertically stacked in two stages.

A cover 16 is provided outside a protruding portion 123 of the base body 121 to cover the Peltier element unit 13 and the sensor substrate 15 from above. The cover 16 is formed into a cylindrical shape, having the lower part opened. The lower end of the cover is attached to the upper surface of the base body 121. The cover 16 has an opening 161 for supply of gas as the substance to be sensed to the excitation electrode 141 disposed on the upper surface of the crystal unit 14.

A space 124 is formed inside the base body 121 and the protruding portion 123. A circuit board 18 provided with the oscillator circuits 17 is housed in the space 124. The oscillator circuits 17 are disposed on the upper and lower surfaces of the circuit board 18, which are opposed to each other. A height dimension of the space 124 is larger than a height dimension of the circuit board 18 including the upper and the lower oscillator circuits 17. For example, a space of approximately 5 mm is formed between the upper surface of the upper oscillator circuit 17 and a ceiling surface of the space 124. A lower side of the circuit board 18 is closed by the back lid 122 of the base portion 12.

A laterally flat casing-shaped connector cover 191 is attached to the lower surface side of the back lid 122 while projecting downward. A connector 19 that allows electrical connection of the circuit board 18 to the outside is housed in the connector cover 191. Connection of the connector 19 to the external power supply unit electrically connects the oscillator circuit 17 and the crystal unit 14 to the outside. Components disposed in a sensing device 1 according to the present disclosure, for example, a crystal unit 4, oscillator circuits 61, 62, and the like will be described later.

In this example, the connector 19 is disposed while projecting toward the lower side of the sensing device 11. The space 124 that houses the oscillator circuits 17 has a large separation distance between the upper surface of the upper oscillator circuit 17 and a ceiling surface 125 of the space 124. The base portion 12 has a relatively large inner space above the circuit board 18. As the base portion 12 is provided with the Peltier element unit 13 via the Peltier base 130, the sensing device 11 has a large dimension in the up-down direction.

As an example of sizes of the above-described sensing device 11, a diameter L11 of the base portion 12 is 35 mm, and a height L12 from the lower end of the connector 19 to the upper end of the cover 16 is 37.25 mm.

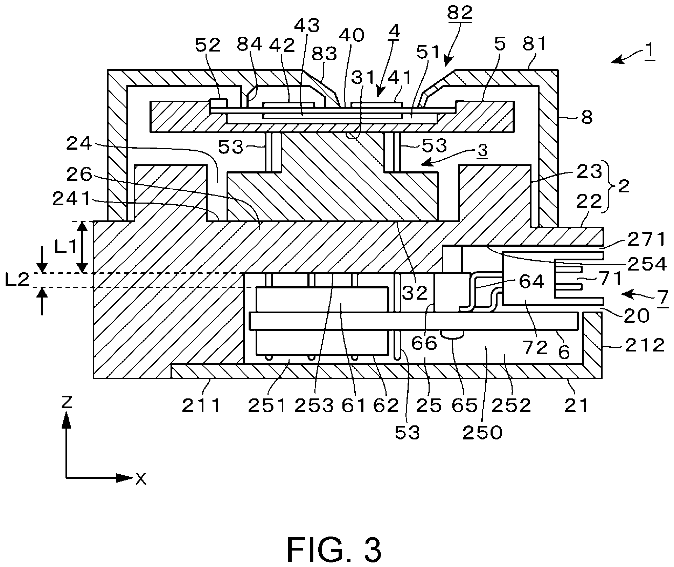

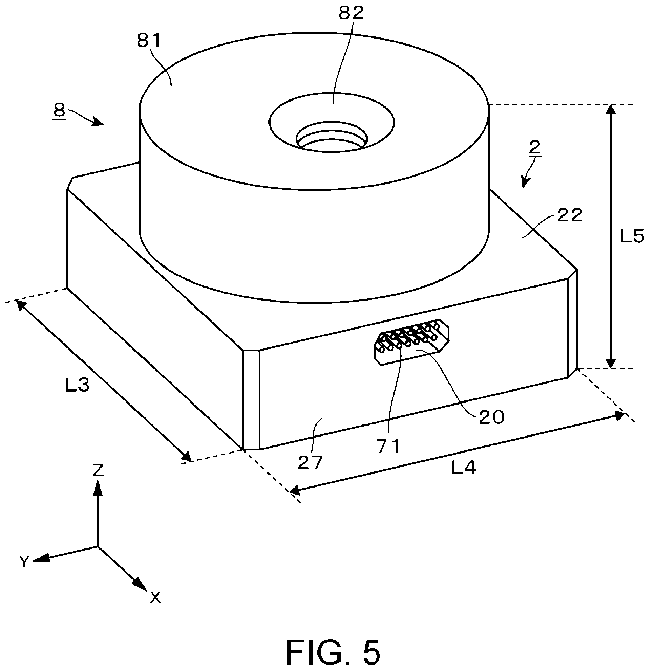

An embodiment of a sensing device 1 as the TQCM according to the present disclosure is described as illustrated in FIGS. 3 to 5. The sensing device 1 is constituted by stacking a Peltier element unit 3 and a sensor substrate 5 that holds a crystal unit 4 as a piezoelectric resonator on the upper surface side of a base body 2. A circuit board 6 is then disposed in a space formed in the lower surface side of the base body 2. The sensing device 1 is different from the sensing device 11 of the comparative configuration in that a connector 7 is directly surface mounted onto the circuit board 6 such that the connector 7 is externally connectable. As illustrated in FIGS. 3 to 5, a left-right direction, a front-rear direction, and an up-down direction to the drawing plane denote an X direction, a Y direction, and a Z direction, respectively. Descriptions will be made on the premise that the right side in the left-right direction to the plane of FIG. 3 denotes one end side, and the left side denotes the other end side.

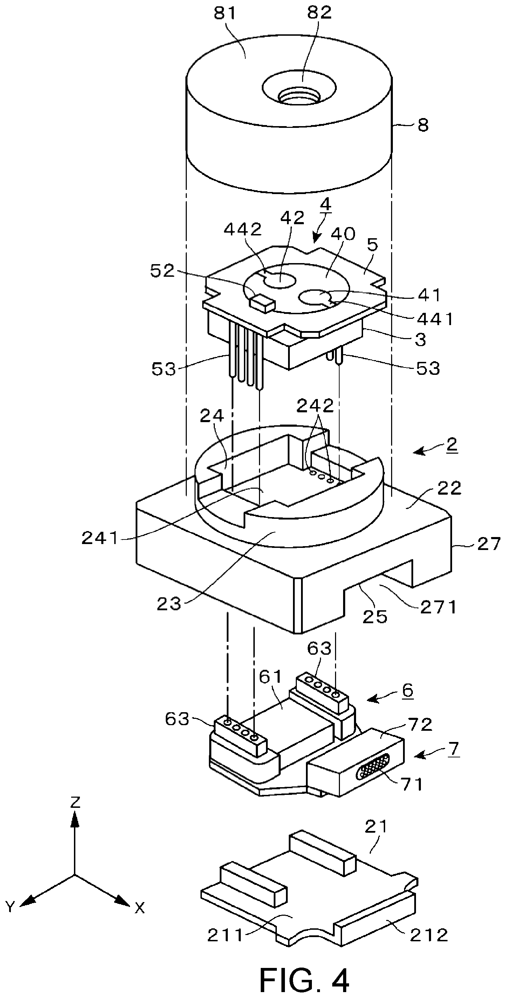

Hereinafter, each component of the sensing device 1 will be specifically described. As illustrated in FIGS. 3 and 4, the base body 2 is constituted of a lower member 22 having a rectangular shape in plan view, and a protruding portion 23 having a circular shape in plan view, which is mounted onto the center of one surface side (upper surface side) of the lower member 22. The base body 2 including the lower member 22 is made of nickel-plated copper to attain high heat transfer property.

A first recessed portion 24 having a substantially rectangular shape in plan view is provided on the upper surface side of the protruding portion 23. When the base body 2 is viewed in plan view, the first recessed portion 24 is formed at the center of the protruding portion 23. A bottom 241 of the first recessed portion 24 has a flat surface, and has holes 242 for receiving insertions of pins 53 to be described later. Those holes 242 are constituted corresponding to the respective positions of the pins 53 (see FIG. 4).

Meanwhile, a second recessed portion 25 is formed in the other surface side (lower surface side) of the lower member 22. As FIG. 3 illustrates, the second recessed portion 25 of the lower member 22 is formed over a range from a region immediately below the first recessed portion 24 to a side surface 27 at one end side of the base body 2 viewed along the left-right direction.

The second recessed portion 25 is formed such that a height dimension of a second region 252 close to the side surface 27 is larger than that of a first region 251 immediately below the first recessed portion 24. As described later, oscillator circuits 61, 62 are disposed in the first region 251, and the connector 7 is disposed in the second region 252. Each of a ceiling surface 253 of the first region 251 and a ceiling surface 254 of the second region 252 is constituted as a flat surface.

As illustrated in FIG. 3, the first recessed portion 24 and the second recessed portion 25, which are positioned opposite to each other are separated by a partition plate 26. An upper surface of the partition plate 26 constitutes the bottom 241 of the first recessed portion 24, and the lower surface of the partition plate 26 constitutes the ceiling surface (the ceiling surface 253 of the first region 251, and the ceiling surface 254 of the second region 252) of the second recessed portion 25. As described above, the partition plate 26 has its thickness varied over a range from the first region 251 to the second region 252. For example, a thickness dimension L1 of the partition plate in the first region 251 is set to a value ranging from 3 mm to 5 mm, for example, 3 mm.

An opening 271 communicated with the second recessed portion 25 is formed in the side surface 27 of the base body 2. The opening 271 combined with a base cover 21 to be described later forms an opening 20 for connecting the connector 7 externally.

The Peltier element unit 3 is disposed in the first recessed portion 24. The Peltier element unit 3 is constituted by vertically stacking large and small Peltier elements each as a small piece having a prismatic shape in plan view. The Peltier element unit 3 having upper and lower flat surfaces is disposed in the first recessed portion 24 such that the lower surface comes in contact with the upper surface of the partition plate 26 (the bottom 241 of the first recessed portion 24). The Peltier element unit 3 is set to be smaller than the sensor substrate 5 in plan view, and disposed such that the upper surface of the Peltier element unit 3 comes in contact with the center of the lower surface of the sensor substrate 5.

The Peltier element unit 3 has a surface (upper surface) in contact with the sensor substrate 5 as a temperature control surface 31 for changing the temperature of the crystal unit 4, and an opposite surface (lower surface) of the temperature control surface 31 as a heat dissipation surface 32. In this example, the upper surface of the Peltier element unit 3 at the upper stage side is constituted as the temperature control surface 31, and the lower surface at the lower stage side is constituted as the heat dissipation surface 32. The whole area at the lower stage side of the Peltier element unit 3 is constituted as the region at the side of the heat dissipation surface. This region is housed in the first recessed portion 24, for example. As illustrated in FIG. 3, the height dimension of the Peltier element unit 3 is larger than the height dimension of the protruding portion 23 (the first recessed portion 24). Accordingly, the temperature control surface 31 as described above is brought into contact with the sensor substrate 5 in the state where the entire part of the Peltier element unit 3 at the upper stage side projects outward from the first recessed portion 24.

A direction of a current supplied to the Peltier element unit 3 is switched such that the temperature control surface 31 is switched to a heating surface or a cooling surface to allow change in the temperature of the crystal unit 4 through the sensor substrate 5. Specifically, when cooling the sensor substrate 5, the temperature control surface 31 of the Peltier element unit 3 becomes the cooling surface, and the heat dissipation surface 32 becomes the heating surface. When heating the sensor substrate 5, the temperature control surface 31 of the Peltier element unit 3 becomes the heating surface, and the heat dissipation surface 32 becomes the cooling surface. The use of the Peltier element unit 3 allows change in the temperature of the crystal unit 4 in the range from -80°C to 125°C, for example.

The sensor substrate 5 is constituted of a Low Temperature Co-fired Ceramics (LTCC), having a substantially rectangular shape in plan view, for example. A recessed portion 51 is formed in the upper surface of the sensor substrate 5. An open edge of the recessed portion 51 supports a peripheral edge of the crystal unit 4. The crystal unit 4 supported by the open peripheral edge is disposed at the center of the sensor substrate 5. A temperature detector 52 for detecting a temperature of the crystal unit 4 is disposed on the upper surface of the sensor substrate 5. As illustrated in FIGS. 3 and 4, the temperature detector 52 is illustrated at the position different from each other for convenience of illustration.

The crystal unit 4 includes a circular plate-shaped crystal piece 40 as an AT-cut piezoelectric piece, for example. As illustrated in FIGS. 3 and 4, a first excitation electrode 41 and a second excitation electrode 42, each made of gold (Au), for example, are disposed on the upper surface side of the crystal piece 40 while being apart from each other in the left-right direction. The first excitation electrode 41 is a reaction electrode that adsorbs a substance to be sensed, and the second excitation electrode 42 is a reference electrode. A third excitation electrode 43 is disposed on the lower surface side of the crystal piece 40 in the region opposed to the first and the second excitation electrodes 41, 42.

The first and the second excitation electrodes 41, 42 are connected to one ends of extraction electrodes 441, 442, respectively. Those extraction electrodes 441, 442, a not illustrated extraction electrode of the third excitation electrode 43, and the temperature detector 52 are electrically connected to upper ends of a plurality of pins 53 each as a rod-shaped conductive member via a not illustrated wiring pattern and a conductive member, which are provided on the sensor substrate 5.

As illustrated in FIG. 4, those pins 53, for example, four pins are disposed at each of both sides of the sensor substrate 5 in the front-rear direction, and arranged along the left-right direction. The respective pins 53 are provided while vertically projecting downward from the lower surface side of the sensor substrate 5. As described above, the Peltier element unit 3 is formed to be smaller than the sensor substrate 5 in plan view. Accordingly, the pin 53 further projects downward through the external region with no interference with the Peltier element unit 3. The pin is inserted through the hole 242 formed in the bottom 241 of the first recessed portion 24 while further projecting downward.

Concerning the base body 2 and the Peltier element unit 3, the lower surface (heat dissipation surface) 32 of the Peltier element unit 3 is adhered to the bottom 241 of the first recessed portion 24 by a metal nanoparticle paste such as an argentum nanoparticle paste. The upper surface (temperature control surface) 31 of the Peltier element unit 3 is fixed to the lower surface of the sensor substrate 5 by the metal nanoparticle paste.

The base body 2 provided with the sensor substrate 5 via the Peltier element unit 3 has the region where the first recessed portion 24 is formed covered with a cover body 8 from above. The Peltier element unit 3 projecting to the outside of the first recessed portion 24 and the sensor substrate 5 are housed in the cover body 8. The cover body 8 is constituted as a raised cylinder having a ceiling. After attaching the base body 2 to the Peltier element unit 3 and the sensor substrate 5, the lower end portion of the cover body 8 is attached to the base body 2 to surround the periphery of the sensor substrate 5, and the side peripheral surface of the protruding portion 23.

A ceiling portion 81 of the cover body 8 has a flat surface. A circular opening 82 in plan view is formed in the ceiling portion 81. The opening 82 is constituted at a position corresponding to the first excitation electrode 41 of the crystal unit 4 such that the substance to be sensed as a gas is allowed to be introduced to the excitation electrode 41. In this example, since the first excitation electrode 41 is the reaction electrode, the region where the first excitation electrode 41 is formed corresponds to a region where the piezoelectric resonator (crystal unit) 4 that adsorbs the substance to be sensed is disposed.

An open edge of the opening 82 in the cover body 8 extends downward to form a cylindrical guide 83 having its diameter of opening gradually decreased as it extends downward. For example, the lower end of the guide 83 is positioned to be slightly apart from the surface of the crystal unit 4 by 0.5 mm, for example. The cover body 8 in this example has a cylindrical wall portion 84 that forms a space surrounding a periphery of the second excitation electrode 42. The lower end of the wall portion 84 is positioned to be slightly apart from the surface of the crystal unit 4.

The configuration of the lower side of the base body 2 of the sensing device 1 will be described. The circuit board 6 is housed in the second recessed portion 25 of the base body 2. The circuit board 6 is a horizontal plate extending in a range across the first region 251 and the second region 252 of the second recessed portion 25. Oscillator circuits 61, 62 are disposed on the upper surface and the lower surface of the circuit board 6, respectively in the region on the other end side (left side to the plane of FIG. 3) when the circuit board 6 is viewed along the left-right direction. Each of the oscillator circuits 61, 62 is constituted of an integrated circuit (IC) as a silicon semiconductor device, and configured to be connected to the crystal unit 4 for oscillation.

Those oscillator circuits 61, 62 are disposed in the first region 251 opposed to the Peltier element unit 3 via the partition plate 26, for example. The circuit board 6 is housed in the second recessed portion 25 such that the upper surface of the oscillator circuit 61 at the upper side is apart from the partition plate 26. Bringing the oscillator circuit 61 into contact with the partition plate 26 may satisfy the requirement of size reduction of the sensing device 1. However, it is preferable to make the oscillator circuit 61 apart from the partition plate in order to suppress the heat dissipation effect from the Peltier element unit 3.

Meanwhile, the thickness dimension L1 of the partition plate in the first region 251 is set to 3 mm, for example, so as to be larger than the thickness dimension of the partition plate in the second region 252. In comparison with the case where the partition plate 26 is formed to have a uniform thickness substantially the same as the thickness of the partition plate in the second region 252, the configuration of the embodiment hardly gives the heat dissipation effect from the Peltier element unit 3 on the lower side. On the other hand, compared with the sensing device 11 according to the comparative configuration, it is difficult for the present configuration to assure the separation distance as large as 5 mm in terms of suppressing the size increase in the sensing device 1. In the embodiment as an exemplified case, a separation distance L2 between the upper surface of the upper oscillator circuit 61 and the lower surface of the partition plate 26 (the ceiling surface 253 of the first region 251) may be set to a value ranging from 0.3 mm to 1.5 mm, for example, 0.5 mm.

The connector 7 for external connection is mounted onto the upper surface of the circuit board 6 in the second region 252. The connector 7 is constituted of a two-piece receptacle 71, 72, for example, which is surface mounted (SMT: Surface Mount Technology) onto the circuit board 6. In other words, the connector 7 is mounted by executing process steps of performing solder printing onto the circuit board 6, installing the receptacle 71, 72 to the circuit board 6, and fixing the receptacle 71, 72 by the solder melting through heating. As illustrated in FIG. 3, the connector 7 is disposed such that the receptacle 71, 72 is directed to the opening 271 formed in the side surface 27 on one end side of the lower member 22 of the base body 2.

The connector 7 is disposed between the circuit board 6 and the partition plate 26 in the second region 252 of the second recessed portion 25 of the base body 2. In the region (the second region 252) in which the connector 7 is disposed, the thickness dimension of the partition plate 26 is smaller than the thickness dimension L1 of the one in the region (the first region 251) in which the oscillator circuits 61, 62 are disposed.

Consideration is made on the case where the height dimension of the receptacle 71 surface mounted onto the upper surface of the circuit board 6 is larger than that of the oscillator circuit 61 disposed on the same surface as represented by an example of the connector 7 as illustrated in FIG. 3. In this case, if the partition plate 26 has the uniform thickness dimension L1 over the entire range, it is necessary to increase the depth of the second recessed portion 25 to assure the space for accommodating the receptacle 71. This may cause enlargement of the sensing device 1.

Formation of the opening 20 for the connector 7 to the side surface 27 of the lower member 22 allows the receptacle 71 to be disposed at a position apart from the region immediately below the Peltier element unit 3 as illustrated in FIG. 3. As the receptacle is disposed at the position apart from the region immediately below the Peltier element unit 3, it is possible to reduce the heat dissipation effect from the Peltier element unit 3. Compared with the oscillator circuit 61, the receptacle 71 itself is insusceptible to an adverse effect of heat. Accordingly, in order to make the sensing device 1 compact, in the second region 252 for accommodating the connector 7, the thickness dimension of the partition plate 26 is reduced to assure the space for the receptacle 71.

As illustrated in the exploded perspective view of FIG. 4, at the upper side of the circuit board 6, two sockets 63 for insertion of the pins 53 are disposed at the front and rear ends of the oscillator circuit 61. Insertion ports are formed in each of the sockets 63 to allow insertions of the plurality of pins 53, for example, the four pins 53. Those insertion ports are arranged along the left-right direction.

The conductive sockets 63 are electrically connected to the respective oscillator circuits 61, 62. Positions of the insertion ports formed in the respective sockets 63 corresponds to the positions of the pins 53 to be inserted into the respective insertion ports. For convenience of illustrations, FIG. 3 omits illustration of the socket 63. One end of a cable 64 is connected to the circuit board 6, and the other end of the cable 64 is connected to the connector 7.

In the above-described configuration, the pins 53 connected to the crystal unit 4 are inserted into the respective insertion ports of the sockets 63 provided on the circuit board 6 through holes 242 of the base body 2. Insertion of the pins 53 into the sockets 63 allows electrical connection between the socket 63 as the electrode connected to the circuit board 6 and the pin 53. As a result, the crystal unit 4 and the temperature detector 52 of the sensor substrate 5, and the circuit board 6 are electrically connected.

The circuit board 6 is fixed to the second recessed portion 25 of the base body 2 using a screw 65, for example. As illustrated in FIG. 3, a reference numeral 66 denotes a member having a screw hole into which the screw 65 is inserted. The circuit board 6 is fixed to the partition plate 26 using a screw in the second region 252, for example.

A base cover 21 for covering the second recessed portion 25 from the lower surface side is provided with the base body 2. The base cover 21 is mounted onto the base body 2 using a not illustrated screw, for example. A space 250 that houses the circuit board 6 is formed between the second recessed portion 25 and the base cover 21. As illustrated in FIGS. 3 and 4, the base cover 21 includes a horizontal plate member 211 formed to be capable of covering the second recessed portion 25 in plan view, and a vertical portion 212 formed by bending one end side of the plate member 211. For example, the thickness dimension of the plate member 211 is set to 1 mm.

The vertical portion 212 is engaged with the opening 271 formed in the side surface 27 on one end side of the base body 2 to form the opening 20 for the connector 7. This allows the receptacle 71 of the connector 7 to be connected to an external plug (not illustrated) via the opening 20.

In the state where the base cover 21 is attached, the separation distance between the lower surface of the oscillator circuit 62 at the lower side of the circuit board 6 and the upper surface of the base cover 21 may be set to a value ranging from 0.3 mm to 1.5 mm, for example, 0.5 mm.

Features of the sensing device 1 according to the embodiment will be described. The sensing device 1 according to the present disclosure is configured to house the connector 7 in the second recessed portion 25 of the base body 2 for external connection from the side surface 27 of the base body 2. Compared with the comparative configuration having the connector 19 projecting downward from the base portion 12, the sensing device 1 has its height dimension reduced to attain size reduction.

The sensing device of the comparative configuration mounts the Peltier element unit 13 onto the base portion 12 via the Peltier base 130. The sensing device 1 according to the present disclosure is provided to house the Peltier element unit 3 in the first recessed portion 24 of the base body 2. The base body 2 and the Peltier base are integrated to allow reduction in the height dimension of the sensing device 1 by the amount equivalent to the thickness dimension of the Peltier base 130.

Focusing on the size reduction of the sensing device 1, the present disclosure intends to reconsider the component of the comparative configuration in regard to the effect of heat on the oscillator circuits 61, 62. Even in the case where the Peltier base 130 is not provided, optimization of the thickness dimension L1 of the partition plate 26 of the base body 2, and the separation distance L2 between the lower surface of the partition plate 26 and the upper surface of the oscillator circuit 61 attains size reduction of the sensing device 1 while suppressing the effect of heat on the oscillator circuits 61, 62 as described above.

Reconsidering the thickness dimension of the base cover 21 makes the sensing device 1 thinner compared with the comparative configuration. An opening diameter of the opening 82 of the cover body 8 is increased to moderate the inclination of the guide 83 such that the separation distance between the sensor substrate 5 and an upper surface 81 (the ceiling portion 81) of the cover body 8 is made smaller compared with the comparative configuration. The above-described improvements intend to make the sensing device 1 further compact.

As the size of the sensing device 1 is reduced by decreasing the number of components or thickness of the configuration, it is possible to further reduce the weight and cost of the sensing device 1. In the comparative configuration, the connector 19 is connected to the circuit board 18 by the cable. The connection is performed by manual soldering. The sensing device 1 according to the present disclosure is configured to perform surface mounting of the connector 7 onto the circuit board 6. The resultant operation process is made simpler than the process of the manual cable connection. Furthermore, the sensing device 1 no longer requires the connector cover 191 necessary for the comparative configuration, thus reducing the number of components.

In the comparative configuration, the Peltier base 130 and the Peltier element unit 13 are bonded, and the bonded configuration is screwed to the base body 12. In the case of the sensing device 1 according to the present disclosure, the Peltier element unit 3 is directly adhered to the base body 2. This may remove the use of the lock bolt required for screwing the Peltier base 130, resulting in the reduced number of components. The cost reduction may also be achieved by decreasing the number of components and the number of man-hours required for work.

Compared with the comparative configuration, the sensing device 1 according to the present disclosure attains reduction in the size, weight, and manufacturing cost. Accordingly, the utilization range of the sensing device 1 that senses the substance to be sensed as a gas may be expanded wider than the conventional application.

As an example of sizes of the sensing device 1, a size L3 of the base body 2 in the left-right direction is 27 mm, a size L4 in the front-rear direction is 29 mm, and a height L5 from the lower end of the base body 2 to the upper end of the cover body 8 is 18 mm. The sizes are smaller than those of the sensing device 11 of the comparative configuration.

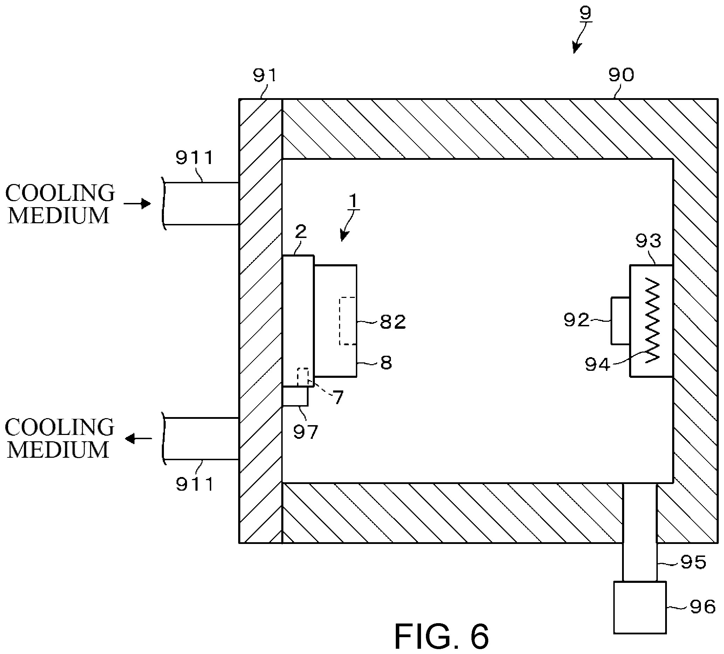

A sensing system 9 using the sensing device 1 will be described as illustrated in FIG. 6. The sensing system 9 includes a vacuum container 90 having one of side surfaces functioning as a cooling unit 91 for cooling the sensing device 1. As illustrated in FIG. 6, the sensing device 1 is installed in the vacuum container 90 by fixing the base body 2 to the cooling unit 91 such that the opening 82 of the cover body 8 is directed sideways. The cooling unit 91 is constituted of a chiller provided with flow passages 911 for circulation of a cooling medium to cool the base body 2 through which the circuit board 6 having the oscillator circuits 61, 62 is cooled.

Because of a high heat transfer property, the base body 2 cooled by the chiller cools the partition plate 26 and the protruding portion 23. Accordingly, the Peltier element unit 3 and the cover body 8, which are in contact with the base body 2 are also cooled to entirely cool the sensing device 1. As the heat of the cooling unit 91 is conducted to the Peltier element unit 3 via the base body 2, it can be said that the base body 2 performs both heat supply and heat dissipation with respect to the Peltier element unit 3. The base body 2 may be configured to perform either heat supply or heat dissipation with respect to the Peltier element unit 3.

A pedestal portion 93 for supporting a sample 92 at a position opposed to the opening 82 of the sensing device 1 is disposed inside the vacuum container 90. This pedestal portion 93 is configured to be heated by a heating mechanism 94 to a predetermined temperature. The vacuum container 90 is connected to a vacuum exhaust mechanism 96 via an exhaust passage 95 such that evacuation is performed to a predetermined degree of vacuum.

When mounting the sensing device 1 onto the cooling unit 91, the receptacle 71 of the connector 7 and a plug provided for a power supply unit 97 are connected to each other in the vacuum container 90. The sensing device 1 is then electrically connected to a not illustrated main body of the sensing system 9. The oscillator circuits 61, 62 are electrically connected to the external main body to allow acquisition of the oscillation frequency of the crystal unit 4. The system is configured to supply current to the Peltier element unit 3, and acquire the temperature of the crystal unit 4, which is detected by the temperature detector 52. Based on the temperature of the crystal unit 4, which is detected by the temperature detector 52, direction of the current to be supplied to the Peltier element unit 3, and the supplied power are adjusted to control the temperature of the crystal unit 4. This makes it possible to raise the temperature of the crystal unit 4 from the predetermined temperature at a predetermined rate.

The sensing system 9 is configured to close the vacuum container 90 after supporting the sample 92 onto the pedestal portion 93, evacuate the vacuum container 90 to the predetermined degree of vacuum, and heat the pedestal portion 93 to 125°C, for example, by the heating mechanism 94. As a result, a gas as the substance to be sensed contained in the sample 92 is sublimated, and discharged into the vacuum container 90. Meanwhile, the cooling medium is supplied to the cooling unit 91, and the Peltier element unit 3 performs temperature control to cool the crystal unit 4 to the temperature of -80°C, for example. This allows the gas generated by heating the sample 92 to flow into the sensing device 1 through the opening 82 for adhesion to the first excitation electrode 41.

Change in mass of the crystal unit 4 varies its oscillation frequency. This allows detection of the substance to be sensed. The Peltier element unit 3 performs the temperature control while continuing acquisition of the oscillation frequency to raise the temperature of the crystal unit 4 at the rate of 1°C/minute, for example. When the temperature rise of the crystal unit 4 desorbs the substance to be sensed adhered to the first excitation electrode 41, the oscillation frequency greatly varies. The user of the sensing system 9 reads the timing of variation in the oscillation frequency from the graph that represents the time-dependent variation in the oscillation frequency. Based on the variation timing, the temperature at which the variation has occurred is determined. Based on the temperature, the specific type of the substance to be sensed may further be determined.

In this example, as the crystal unit 4 is provided with the second excitation electrode 42 (the reference electrode), the oscillation frequency F1 at the side of the reaction electrode 41, and the oscillation frequency F2 at the side of the reference electrode 42 are measured. The difference between the oscillation frequencies F1 and F2 is acquired. With reference to a relationship between the preliminarily acquired difference and the amount (mass) of the substance to be sensed, it is possible to detect the amount of the substance to be sensed corresponding to the difference.

The sensing device 1 is used in the vacuum atmosphere of the sensing system 9. The space 250 between the second recessed portion 25 and the base cover 21 for housing the circuit board 6 is brought into the vacuum atmosphere. The oscillator circuit 61 disposed apart from the partition plate 26 is vacuum insulated from the partition plate 26. Accordingly, even if the separation distance L2 between the oscillator circuit 61 and the partition plate 26 is relatively short, specifically, ranging from 0.3 mm to 1.5 mm, the heat dissipation effect given by the partition plate 26 is originally small. Meanwhile, the partition plate 26 itself is promptly cooled because of heat conduction from the cooling unit 91. Accordingly, the heat effect given by the partition plate 26 is suppressed. Under the above-described circumstances, significant heat effect on the oscillator circuit 61 may be avoided even if the separation distance L2 is set to the small value for the purpose of reducing the size of the sensing device 1.

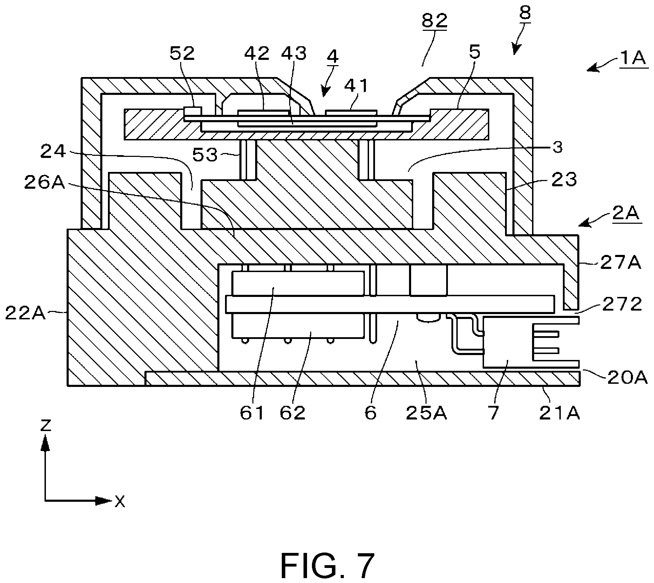

FIG. 7 illustrates another example of the sensing device 1 according to the present disclosure. This drawing illustrates an example that the connector 7 is disposed below the lower surface of the circuit board 6. In this example, a base body 2A includes a lower member 22A and the protruding portion 23. The configuration of a section at the upper side of a partition plate 26A is similar to the configuration according to the embodiment as described above.

Meanwhile, the partition plate 26A may be configured to have a uniform thickness dimension, for example. The circuit board 6 provided with the oscillator circuits 61, 62 is disposed in a second recessed portion 25A formed below the partition plate 26A. The connector 7 is surface mounted onto the lower surface of the circuit board 6.

The second recessed portion 25A is covered with a base cover 21A from the lower side. An opening 20A for the connector 7 is then formed between the base cover 21A and an opening 272 formed in a side surface 27A on one end side of the base body 2A.

In this example, the thickness dimension of the partition plate 26A, for example, is adjusted to make the height dimension of the lower member 22A of the base body 2A equivalent to that of the lower member 22 in the embodiment as illustrated in FIGS. 3 and 4. This allows the sensing device 1 to be compact.

The device as described in the embodiment is configured to have the receptacle 71 with the two-piece connector disposed below the circuit board 6 for connection to the external plug. Meanwhile, the device may also be configured to surface mount the plug of the two-piece connector onto the circuit board 6 for connection to the receptacle provided outside. The embodiment is configured to have the first excitation electrode 41 as the reaction electrode and the second excitation electrode 42 as the reference electrode disposed on one surface side of the crystal unit 4. It is also possible to provide only the excitation electrode as the reaction electrode on one surface side of the crystal unit 4.

The principles, preferred embodiment and mode of operation of the present invention have been described in the foregoing specification. However, the invention which is intended to be protected is not to be construed as limited to the particular embodiments disclosed. Further, the embodiments described herein are to be regarded as illustrative rather than restrictive. Variations and changes may be made by others, and equivalents employed, without departing from the spirit of the present invention. Accordingly, it is expressly intended that all such variations, changes and equivalents which fall within the spirit and scope of the present invention as defined in the claims, be embraced thereby.

### Description of Reference Numerals

1, 11: sensing device
2, 2A: base body
3, 13: Peltier element unit
4, 14: crystal unit
5, 15: sensor substrate
6, 18: circuit board
7, 19: connector
8: cover body
9: sensing system
12: base body
16: cover
17, 61, 62: oscillator circuit
20, 20A, 82, 161: opening
21, 21A: base cover
22, 22A: lower member
23, 123: protruding portion
24: first recessed portion
25, 25A: second recessed portion
26, 26A: partition plate
27, 27A: side surface
31: temperature control surface
32: heat dissipation surface
40: crystal piece
41, 42, 43, 141, 142: excitation electrode
51: recessed portion
52: temperature detector
53, 151: pin
63: socket
64: cable
65: screw
66: a member having a screw hole into which the screw is inserted
71, 72: receptacle
81: upper surface (ceiling portion)
83: guide
84: wall portion
90: vacuum container
91: cooling unit
92: sample
93: pedestal portion
94: heating mechanism
95: exhaust passage
96: vacuum exhaust mechanism
97: power supply unit
121: base body
122: back lid
124, 250: space
125, 253, 254: ceiling surface
130: Peltier base
131, 132: Peltier element
191: connector cover
211: plate member
212: vertical portion
241: bottom
242: hole
251: first region
252: second region
271, 272: opening
441, 442: extraction electrode
911: flow passage
L1: thickness dimension
L2: separation distance
L3: a size of the base body in the left-right direction
L4: a size of the base body in the front-rear direction
L5, L12: height
L11: diameter

## Claims

1. A sensing device (1, 1A) that adsorbs a substance to be sensed as a gas to a piezoelectric resonator (4), and desorb the substance to be sensed by changing a temperature of the piezoelectric resonator (4) to sense the substance to be sensed based on a relationship between a variation in an oscillation frequency of the piezoelectric resonator (4) and the temperature, the sensing device (1, 1A) comprising:
a sensor substrate (5) that holds the piezoelectric resonator (4);
a Peltier element unit (3) in contact with the sensor substrate (5), the Peltier element unit (3) including a temperature control surface (31) for changing the temperature of the piezoelectric resonator (4) and a heat dissipation surface (32) opposite to the temperature control surface (31);
a circuit board (6) that is electrically connected to the piezoelectric resonator (4) and includes an oscillator circuit (61, 62) installed for oscillating the piezoelectric resonator (4) and a connector (7) that is surface-mounted for an external connection;
a base body (2, 2A) that includes a first recessed portion (24) for housing a region of the Peltier element unit (3) at a side of the heat dissipation surface (32), a second recessed portion (25, 25A) for housing the circuit board (6), and a partition plate (26, 26A) interposed between the first recessed portion (24) and the second recessed portion (25, 25A);
a cover body (8) that houses the base body (2, 2A) in a region where the first recessed portion (24) is formed, the Peltier element unit (3) projecting outside the first recessed portion (24), and the sensor substrate (5), the cover body (8) having an opening (82) formed at a position corresponding to a region where the piezoelectric resonator (4) is disposed for adsorption of the substance to be sensed; and
a base cover (21, 21A) that covers the second recessed portion (25, 25A),
wherein
the connector (7) is externally connected from a side surface side of a region where the second recessed portion (25, 25A) of the base body (2, 2A) is formed.

2. The sensing device (1, 1A) according to claim 1, wherein
the Peltier element unit (3) is housed in the first recessed portion (24) to bring the heat dissipation surface (32) into contact with the partition plate (26, 26A); and
the circuit board (6) is housed in the second recessed portion (25, 25A) to cause the oscillator circuit (61, 62) to be apart from the partition plate (26, 26A).

3. The sensing device (1, 1A) according to claim 2, wherein
a separation distance between the oscillator circuit (61, 62) and the partition plate (26, 26A) is in a range from 0.3 mm to 1.5 mm.

4. The sensing device (1, 1A) according to claim 2, wherein
the sensing device (1, 1A) is used in a vacuum atmosphere; and
a space between the second recessed portion (25, 25A) and the base cover (21, 21A) for housing the circuit board (6) brought into the vacuum atmosphere causes the oscillator circuit (61, 62) disposed apart from the partition plate (26, 26A) to be vacuum insulated from the partition plate (26, 26A).

5. The sensing device (1, 1A) according to claim 1, wherein
the connector (7) is disposed between the partition plate (26, 26A) and the circuit board (6);
a thickness dimension of the partition plate (26, 26A) in a region where the oscillator circuit (61, 62) is placed is larger than a thickness dimension of the partition plate (26, 26A) in a region where the connector (7) is placed.

6. The sensing device (1, 1A) according to claim 4, wherein
a thickness dimension of the partition plate (26, 26A) in a region where the oscillator circuit (61, 62) is placed is in a range from 3 mm to 5 mm.
